# EUROPEAN PATENT SPECIFICATION

(11) **EP 2 313 007 B1**
(45) Date of publication and mention of the grant of the patent: **28.03.2012**
(21) Application number: 09785427.7
(22) Date of filing: 31.07.2009
(51) Int. Cl.: A61B 17/00, A61B 1/005, A61B 17/32, A61M 25/00

(54) **ARTICULATING SHAFT FOR AN ENDOSCOPIC SURGICAL INSTRUMENT**
GELENKSCHAFT FÜR EIN ENDOSKOPISCHES OPERATIONSINSTRUMENT
TIGE ARTICULÉE POUR UN INSTRUMENT CHIRUGICAL ENDOSCOPIQUE

(30) Priority: 31.07.2008 GB 0813990; 18.03.2009 GB 0904602; 09.04.2009 GB 0906184
(43) Date of publication of application: 27.04.2011
(73) Proprietor: Surgical Innovations Limited, West Yorkshire LS16 6QZ (GB)
(72) Inventor: MAXWELL, Vhairi, Yorkshire LS16 6QZ (GB); WHITE, Michael, Yorkshire LS16 6QZ (GB); McMAHON, Michael, Yorkshire LS16 6QZ (GB)
(74) Representative: Sherrard-Smith, Hugh
(86) International application number: PCT/GB2009/050953
(87) International publication number: WO 2010/013060

(56) References cited:
- EP-A- 1 584 293
- EP-A- 1 836 986
- WO-A-2005/094661
- WO-A-2007/136829
- US-A1- 2006 178 556

## Description

The present invention relates to endoscopic surgical instruments and elongate portions for use in such instruments. The instruments are particularly, but not exclusively relevant to instruments that are able to snare sutures.

Endoscopic surgical instruments are widely, though not exclusively, used in abdominal surgery. In abdominal surgery it is possible for a surgeon to examine or operate on abdominal organs without the physical insertion of the surgeons fingers. This is done by making a hole through the skin and other tissues in order to enter a body cavity, blowing inert gas under pressure into the abdominal cavity through that hole to create space, and inserting an endoscopic camera through that opening so that the surgeon can view the organs on a remote monitor. Other tubes are then placed in the abdominal wall (usually of 5 - 11 mm diameter), which are sealed to prevent or restrict the egress of the inert gas, and surgical instruments can then be inserted through these tubes. The surgical instruments can either be for operating on the organs or for pushing organs out of the way, and the surgeon is able manipulate then from outside the body,

EP 0 623 004 (McMahon and Moran) discloses an endoscopic surgical instrument comprising an elongate portion made from a plurality of separate segments. The segments are threaded onto two parallel wires which are located towards opposed sides of the segments. The surgeon operates the endoscopic surgical instrument by operating a handle attached to the elongate portion. By tensioning the wires the surgeon is able to cause the segments to bend towards each other. The two wires are pulled simultaneously to prevent misalignment of the segment faces, As the wires are further tensioned, faces on adjacent segments abut to create a rigid hook.

There are many other publications that also disclose endoscopic surgical instruments that include a plurality of segments that can be manipulated. However, in all these documents the segments are either separate from each other,or are pivotally connected to each other. These include US2,079,233; US3,109,286; US3,642,352; US3,799,151; US3,958,576; US4,226,228; US4,239,036; US4,483,562; US4,754,909; US4,950,273; US4,982,727; US5,035,248; US5,152,779 DE 4 021 153; EP1 815 801; DE4 243 715; DE1960 8809; DE1992 8272; EP1 836 949; FR2 713 492; US4,353,358; US5,501,654; US5,669,926 and EP1 864 625.

A problem with such endoscopic surgical instruments is the construction and manufacture of the elongate portion made from a plurality of segments. In this respect the segments have to be connected to each other or threaded like beads onto a string. This is time consuming. Further, manufacturing tolerances can result in different performances from different instruments. Clearly this is not desirable in surgical operations where the surgeon trusts in repeatable performance from different instruments. Moreover, the instruments risk breaking up/disassembling whilst inside the body cavity. For instance, the wires could snap due to over tensioning and the segments unthreaded or, the segments could crack and/or break due to stress cracking caused by the segments abutting during repeated and/or over tensioning of the wires.

Various attempts have been made to attempt to overcome the above problems. These include DE 19928272, EP 1836949, US 2007/0049800, US 4580551, WO 2008/033589, WO 2005/120326, WO 2005/094661, WO 02/053221, US 2003/0135204 and WO 00/76570. However, these devices are difficult and time consuming to make. In addition where segments are integrally moulded the mould is complicated and it is difficult to obtain repeated evenly formed mouldings.

Where such prior publications include a control member to alter the orientation of mouldings these can be difficult to thread through the sections. In addition, it can be difficult to detect a faulty or weak portion of a control member where it is threaded through a section.

There are also patent publications that relate to the gripping of sutures or needles by the instruments during endoscopic surgery. These include US5,951,587; US1,445,348; US3,408,554; US5,304,185; US5,413,583; US5,601,575; US5,817,111 and US5,449,366. However, where these publications attempt to catch a suture, they are difficult to operate and manipulate. Further, the suture may snag on other parts of the instrument. In addition they can tend to catch blood vessels when advancing or retreating though body tissue.

It is an object of the present invention to attempt to overcome at least one of the above or other problems.

The present invention is defined in the independent claims 1 and 11 and the dependent claims.

WO 2005/094661 discloses an elongated portion according to the preamble of claims 1 and 11.

According to the present invention an elongate portion suitable for use as part of a surgical instrument includes at least two adjacent integrally formed sections movable relative to each other from a first position to a second position whereby the relative direction in which a part of the elongate portion extends when compared to another part of the elongate portion is altered by a control member that extends from a proximal end of the instrument first through an opening in one section and then through an opening in another section, the opening is exposed along the complete elongate extent of each section, characterised in that the opening is at one side of the instrument at one elongate location of at least one section and at the other side at a different elongate extend of that section.

According to another aspect of the present invention an elongate portion suitable for use as part of a surgical instrument includes at least two adjacent integrally formed sections movable relative to each other from a first position to a second position whereby the relative direction in which a part of the elongate portion extends when compared to another part of the elongate portion is arranged to be altered wherein, an operative tool is integrally formed with the at least two adjacent sections.

The operative tool may comprise a catcher.

A flexible portion may connect the at least two adjacent portions which flexible portion is integrally formed with the adjacent sections. The flexible portion may extend from one side of the instrument to the other. The flexible portion may be of reduced width when viewed along the elongate extent in at least one direction. The flexible portion may extend to opposed sides of the adjacent sections. The flexible portion may extend across the centre of the elongate extent of the instrument. The flexible portion may assist in returning the adjacent sections to a predetermined relative elongate extent when offset from the predetermined extent.

The control member may extend through an opening in at least one section.

The control member may be arranged to be at a different location to the flexible member when viewed along the elongate extent.

The sections may be rigid.

The sections may be enclosed by a sleeve.

The elongate portion may be arranged, in use, to be inserted into a body cavity.

According to a further aspect of the present invention an elongate portion for use as part of a surgical instrument includes a recess in the exterior of the portion leading to a catcher located inwardly from the exterior of the portion, the recess having a first region on the distal side that extends distally and outwardly towards the distal end and a second region on the proximal side that extends proximally and outwardly towards the proximal end.

The elongate portion may include at least two adjacent integrally formed sections that are movable relative to each other. The sections may be movable relative to each other from a first position to a second position whereby the relative direction in which a part of the elongate portion extends when compared to another part of the elongate portion is altered. The alteration may be effected by a control member that extends through an opening in one section and then an opening in another section. The opening may be exposed along the complete elongate extent of each section.

The first region may extend distally and outwardly over the complete extent of the recess from the catcher to the exterior of the portion.

The second region may extend distally and outwardly over the complete extent of the recess from the catcher to the exterior of the portion.

The recess may extend across the portion from one side to the other.

The outermost portion of the recess in a direction parallel to the elongate extent of the portion may be the same distance from the centre of the elongate portion at both the proximal and distal region of the recess at at least one location and preferably at all locations.

The catcher may extend across the portion from one side to the other. The outermost portion of the catcher in a direction parallel to the elongate extent of the portion may be the same distance from the centre of the elongate portion at both the proximal and distal regions of the catcher at at least one side and preferably at both sides.

The first region may include two surfaces that each extend from a central area of the first region to a different side, the surfaces extending outwardly and distally, the surfaces also diverging from each other from the central area. The surfaces may be planar. The surfaces may include an angle of less than 180° at the central area which angle may be 140°. The surfaces may comprise a chamfer.

The second region may include two surfaces that can extend from a central area of the second region to a different side of the first region to a different side, the surfaces extending outwardly and proximally, the surfaces also diverging from each other from the central area. The surfaces may be planar. The surfaces may include an angle of less than 180° at the central area which angle may be 140°. The surfaces may comprise a chamfer.

The catcher may comprise a slot that leads from a closed end towards the recess.

The slot may include a region which is narrower than the remainder of the slot at a location spaced from the closed end which narrow region may be located at the end region of the slot furthest from the closed end.

The second region may include two surfaces that each extend from a central area of the second region to a different side, the surfaces extending outwardly and distally, the surfaces also diverging from each other from the central area. The surfaces may be planar. The surfaces may include an angle of less than 180° at the central area which angle may be 140°. The surfaces may comprise a chamfer.

The slot may extend proximally and downwardly towards the closed end.

The present invention also includes a surgical instrument including an elongate portion as herein referred to.

Not forming part of the invention is a method of operating a surgical instrument including a recess leading to a catcher, the recess having a first region on the distal side that extends distally and outwardly towards the distal end and a second region that extends proximally and outwardly towards the proximal end comprises pushing the instrument through body tissue with tissue being pushed outwardly by the second region and retracting the instrument to push tissue outwardly by the first region.

The method not forming part of the invention may also comprise catching a surgical item such as a suture by causing the item to be squeezed past a reduced area of the catcher. The method may comprise a surgeon registering the passage of the item past the reduced area by feeling such passage from a remote end region of the instrument. The method may comprise causing a force to be exerted relative to the item and the catcher urging the catcher to be withdrawn from the catcher without such withdrawing taking place. The method may comprise a surgeon registering the passage of an item past the reduced area to release the item from the catcher by the surgeon feeling such passage.

A method of operating a surgical instrument not forming part of the invention as herein referred to comprises altering the elongate extent of at least part of the elongate portion by causing movement of two adjacent sections from the first to the second position.

The surgical method not forming part of the invention may comprise using an elongate portion and may comprise inserting the elongate portion into a body cavity and then causing two adjacent sections that are integrally formed to move from a first position to a second position whereby the relative direction in which a part of the elongate portion extends when compared to another part of the elongate portion is altered.

According to a second aspect there is provided an endoscopic surgical instrument having a catching means such as a gripping means, for example to catch or grip a suture. The gripping means may comprise a clamp. The clamp may be operable by pushing and/ or pulling a control. For instance the control may be a wire. The wire may be the same or separate to that used to articulate the movement of the endoscopic instrument.

The present invention may be carried into practice in various ways but one embodiment will now be described by way of examples with reference to the accompanying drawings, in which:-
Figures 1A, 1B and 1C show a distal end of an endoscopic surgical instrument from various angles;
Figures 2A, 2B and 2 C show in sequence the snaring of a gastric band or suture;
Figures 3A and 3B are enlarged end views of the end of a catcher of the instrument that may be used with any embodiment;
Figures 4A, 4B and 4C and Figure 5 show alternative catcher arrangements;
Figures 6 and 7 are side views of the distal end of the instrument with an articulated section being exposed in Figure 6;
Figure 8 is an enlarged sectional view along the line v-v of Figure 6;
Figures 9 and 10 are side and plan views respectively of a distal part of the instrument according to an alternative embodiment;
Figure 11 is a sectional view along the line viii-viii of Figure 9;
Figure 12 is an exploded view of the instrument, without the control that can cause the segments to be articulated;
Figure 13 is an enlarged view of the distal end of Figure 12;
Figure 14 is an enlarged view of the proximal end of Figure 12;
Figures 15 and 16 are cross sectional views through the assembled proximal end of the instrument;
Figure 17 is a top view of a control mechanism to cause the articulation of the instrument, and
Figure 18 is a perspective view through the parts of a mould, in a spaced position, which are used to form the articulated section;

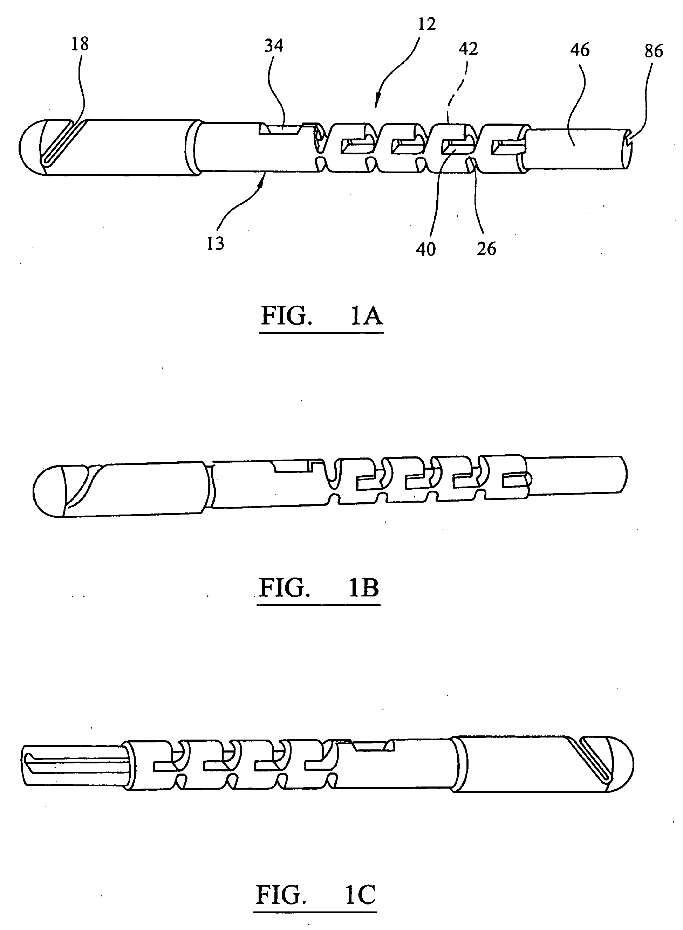

Figures 1A, 1B and 1C show a distal end of an endoscopic surgical instrument 12. The distal end comprises an articulated section 13 formed from a single piece moulding. The articulate section 13 comprises a series of sections 24 that can be caused to move towards each other when the instrument is activated such that the articulated section 13 forms a curve or shape rather than being substantially elongate when unactivated. A catcher 18 is formed at the end of the articulated section 13. An end 46 of the moulding is of reduced external diameter in order to fit within a tube 46 of another part of the instrument

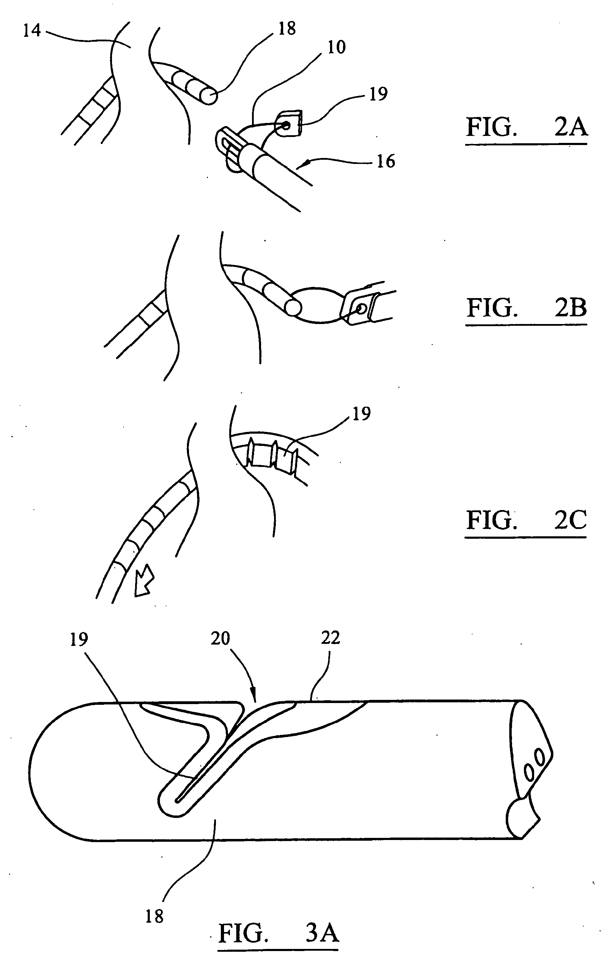

Figures 2A, 2B and 2C show the sequence of snaring a suture 10. The endoscopic instrument 12 is inserted through the body tissue and is then articulated behind a section of stomach 14. A further instrument 16 then offers up the suture 10 in the form of a loop to the catcher 18 of the instrument 12. Once caught, as shown in Figure 1B, the curve on the end of the instrument can be reduced and the instrument 10 can be retracted, possibly at the same time, to bring the suture 16 back which suture, in turn, brings the gastric band 19 partially around the stomach 14.

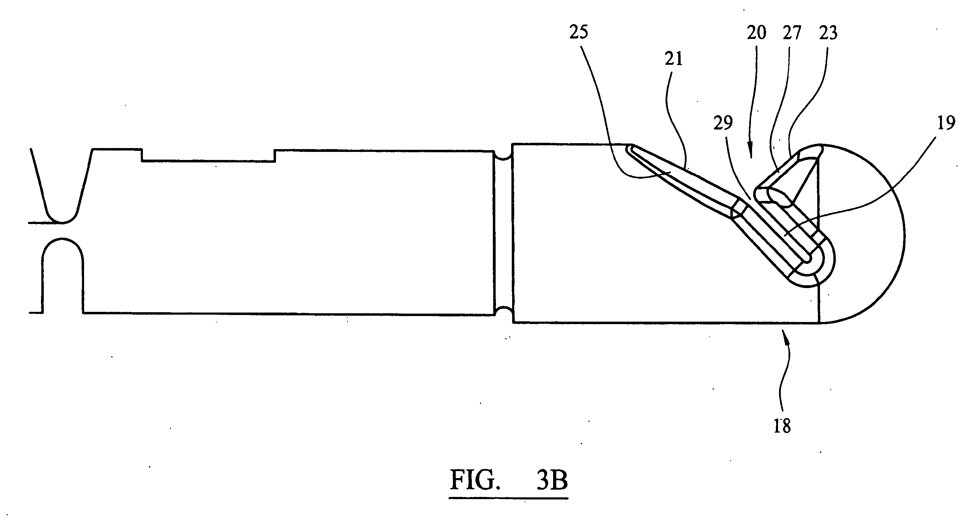

As shown in Figures 3A and 3B, the distal end of the instrument 12 includes the catcher 18. The catcher comprises a slot 19 having an opening 20 on one side of the instrument with the slot extending across the instrument and in a direction angled towards the distal end of the instrument. As the slot in Figure 3A extends away from the opening 20 the slot decreases in width to ensure that the suture 10 is firmly gripped. The walls of the slots may be lined with an elastomeric material such as neoprene 22 to ensure an even better grip on the suture. The elastomeric lining within the slot adds grip to prevent accidental dropping of the suture when high forces are put on the suture to pull the band through, for example, a retro gastric tunnel.

Figure 3B differs from Figure 3A in the following respects. The rear ridge 21 and the forward ridge 23 that lead away from the opening towards the outer side of the instrument extend respectively, upwardly and rearwardly and upwardly and forwardly. In addition the facets 25 and 27 extend to either side of the ridges 21 and 23 (only one of which facets are shown) which facets have an inclusive angle of less than 180° which is in the region of 140°.

The ridges 21 and 23 and the facets 25 and 27 assist in preventing blood vessels and other tissue being snagged by the slot 19 as the body parts will ride over the ridges and edges where the instrument is advanced or retracted with the facets helping in preventing snagging even if the instrument is not advanced in a linear direction. When a suture is being caught the facets assist in guiding the suture into the opening 20.

In addition, in Figure 3B, the slot includes a narrowed nip 29 at the entrance. The instrument can be manipulated to locate a suture at the end of the slot with the instrument then being pulled (or the suture pulled) to cause the suture to move past the nip, possibly causing a small compression of the nip on passing the nip, to enter the main extent of the slot 19. The surgeon may possibly feel a click as the suture passes the nip to alert the surgeon to the successful entrapment.

When a suture is trapped the surgeon can advance or retract the instrument even in a direction that causes a force to be exerted on the suture to leave the slot providing that the force is not sufficient to enable the suture to pass the nip.

Should the suture leave the slot, on purpose or accidentally, then again it is possible that the surgeon will feel a click as the suture passes the nip.

Whilst figures in the specification describe the catchers or operational parts at the end of the instrument as being in an articulated part of the instrument or on an integrally moulded part of the instrument it is possible that the catchers or operational parts could be separately formed from the articulated section or could be located on an instrument without an articulation section.

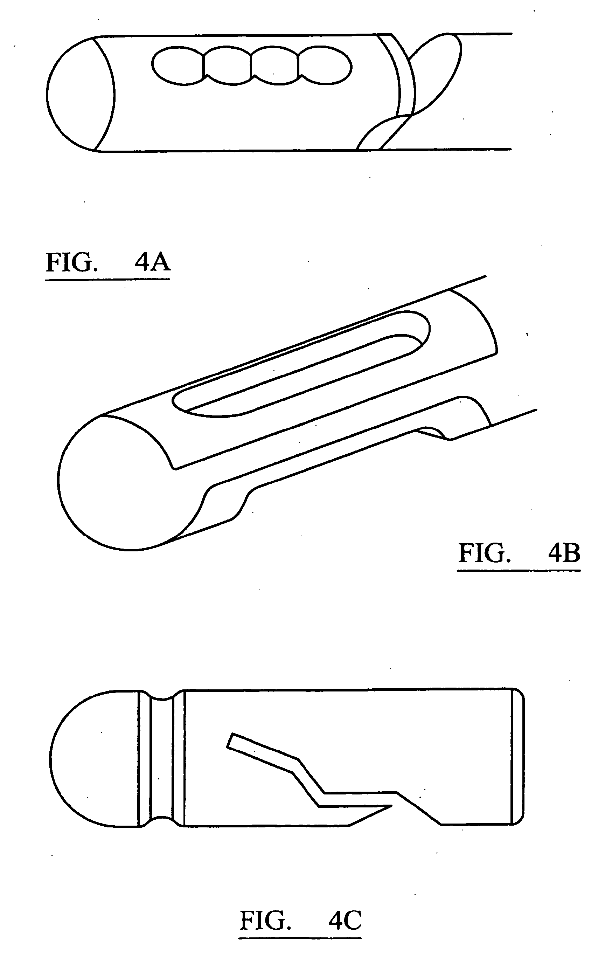

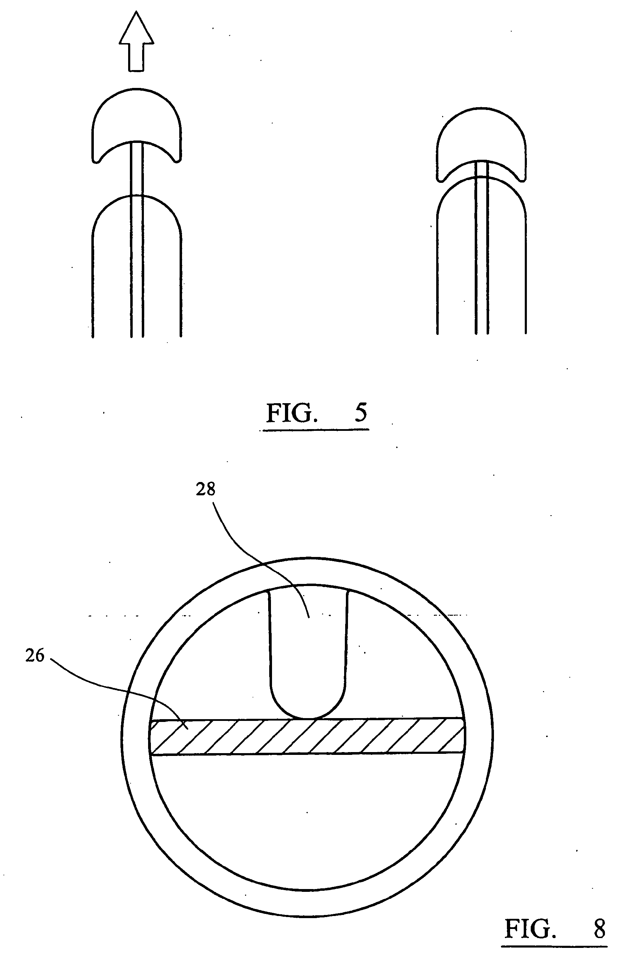

Whilst the catcher has been described in relation to snaring a suture it will be appreciated that it could easily be used or adapted to pull surgical tape and other such components. For example, figures 4A, 4B and 4C show various slot/opening profiles suitable for gripping a gastric band either directly or indirectly through a suture attached to the band. Figure 5 shows a further alternative distal end wherein the distal end includes a tip that can be actuated away from and towards the end face of the single piece articulated section by pushing / pulling a cable. The suture is therefore able to be clamped between a concave profile of the tip and convex face of the one piece section.

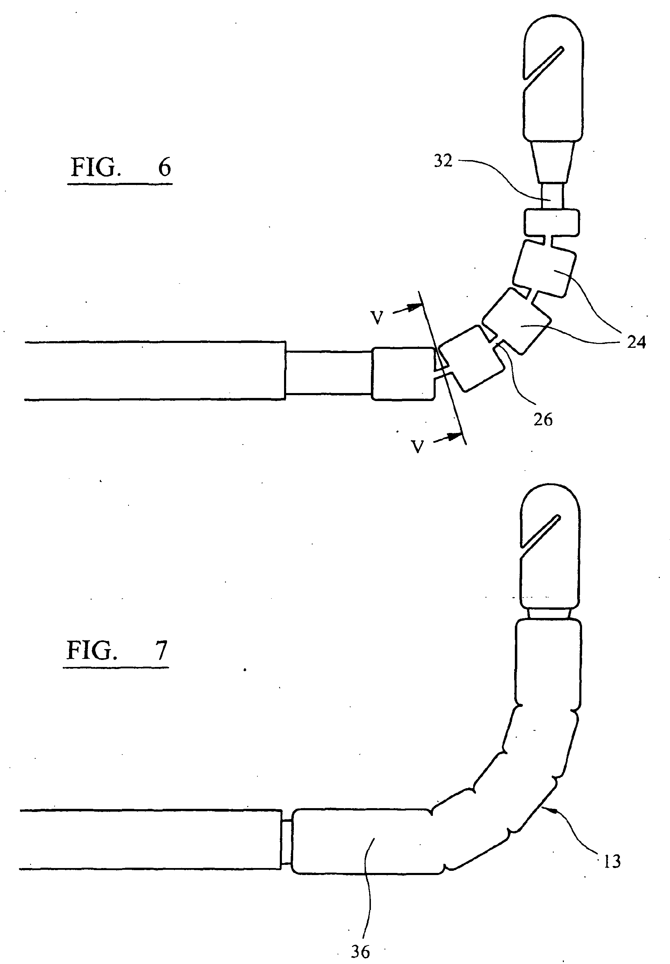

As shown in Figure 6 the articulated section of the distal end is made up of a series of sections 24. The sections 24 and the catcher 18 are integrally moulded such as by injection moulding polymer, such as polypropylene. Each section 24 has a circular periphery.

Each section is connected to an adjacent section by a hinge 26 that extends across the centre of the section from one side to the other. Each hinge has the same angular relationship to the elongate extent of the instrument.

The sections each include an opening 28 through which an articulation operator 30 comprising a stainless steel micro cable can extend. The end of the operator 30 includes an enlarged head 32 comprising a crimped end of the cable that sits in a recess 34 located at the end of the most distal segment.

In order to articulate the distal end, tension is applied to the operator 30 which pulls the sections to the position shown. It will be appreciated that by varying the tension of the operator the length of the sections along one side is varied. Accordingly the sections in this or other embodiments can occupy any curvature. The sections in this or other embodiment may also articulate in the opposite direction by pushing the operator 30 to increase the length of the sections on that side such that the gaps between the sections on the other side are reduced. The hinges 26 may assist in bringing the sections back to a predetermined position as a result of the natural flexure of the hinge.

When the sections are activated to the desired limit position, a restriction means to stop the sections from moving further is required. In the prior art, the restriction means comprises the adjacent faces of the section from abutting. Whilst this is a possibility it is advantageous that they do not abut. That is because, when catching a suture, a small amount of flexure may be desirable. That flexure may be caused by tension in the suture being exerted as the suture slides along the instrument, which tension is released when the suture enters the opening 20 of the catcher. Moreover, if the sections avoid abutment the risk of cracking/breaking up of the sections is reduced. Accordingly, the sections are delimited before they abut by limiting the tension that can be applied to the control member 30 as herein described.

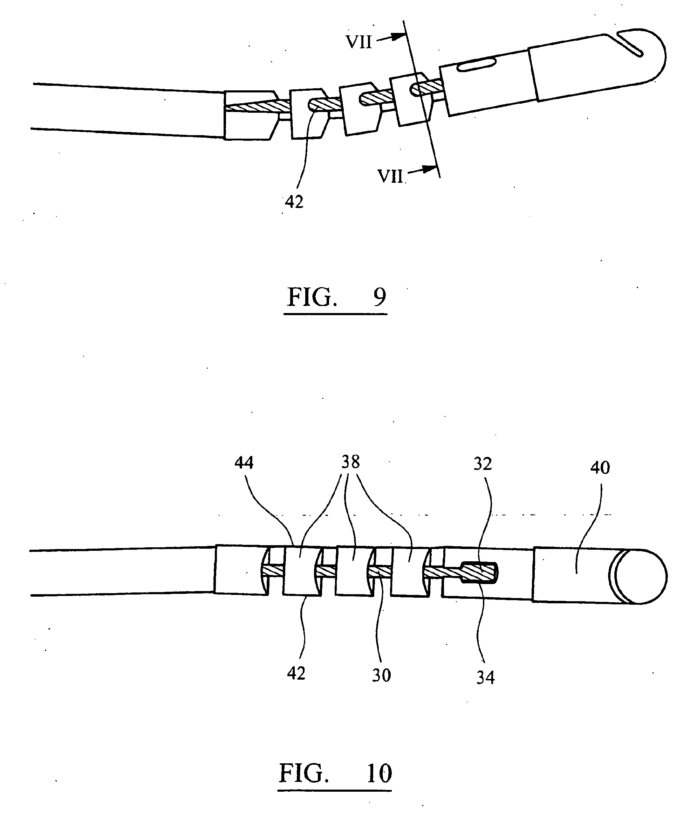

Figure 7 shows the articulated section 13 of the instrument, excluding the catcher, enveloped by a heat shrunk sleeve 36. The sleeve 36 prevents any parts that may come loose from leaving the instrument. The sleeve 36 prevents tissue and the suture from being caught in the gaps between the sections. Accordingly, the sleeve prevents tissue from becoming trapped in the instrument. The sleeve 36 may be used with the embodiment disclosed in Figures 9 and 10.

In Figures 9 and 10, the sections 38 and the catcher 40 are also integrally moulded such as by injection moulding polymer. The only difference between Figures 9 and 10 and the previously described embodiment is in the form of the sections 38. As shown in Figures 9, 10 and 11, each section includes a slot 42 along one side for half the length of the section and a slot 44 along the other half in order to locate the operator 30. This arrangement facilitates injection moulding of the articulated section.

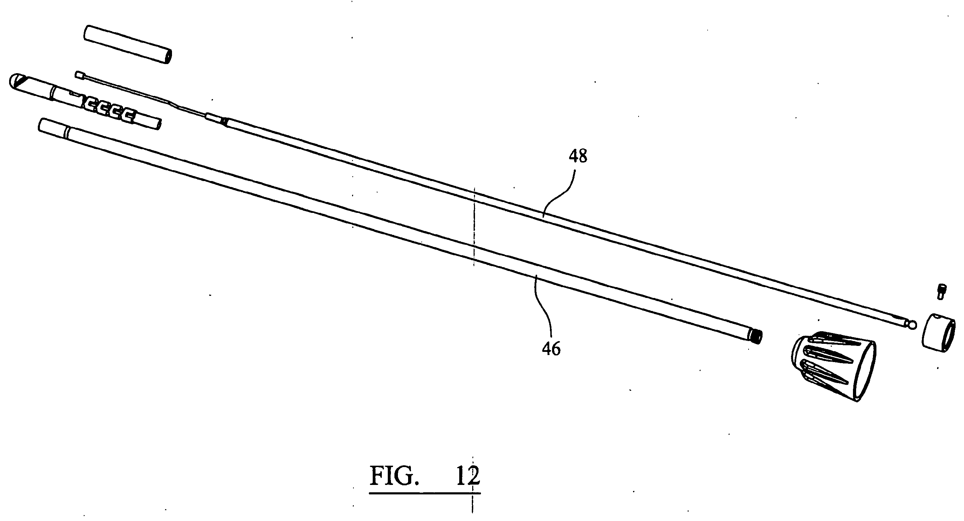

Referring now to Figures 12 to 14 these show, in enlarged view, the details of the sections 38 and catcher 40 together with the control 30 and how these parts are connected to the remainder of the instrument.

The trailing section 38 abuts a hollow member comprising the tube 46. The control 30 comprising the cable is attached to the distal end of a rod 48 that is located within the tube and is able to slide along the tube.

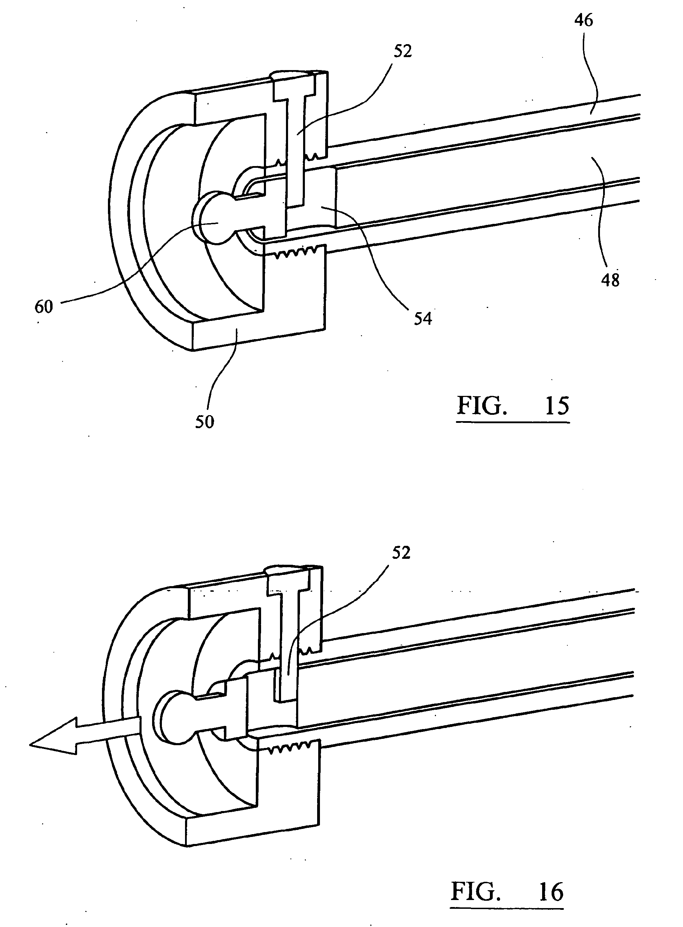

As shown in Figures 15 and 16, collar 50 surrounds the end region of the tube 46 and is fast therewith. The collar has a detent 52 that passes through the collar and the tube 46 and extends into a slot 54 of the rod 48. Relative axial movement of the rod to the tube is restricted by abutment of either end of the slot 54 with the detent 52. In this way curvature of the sections in either direction is also restricted, and it will be appreciated that this restriction prevents the sections from abutting as previously mentioned.

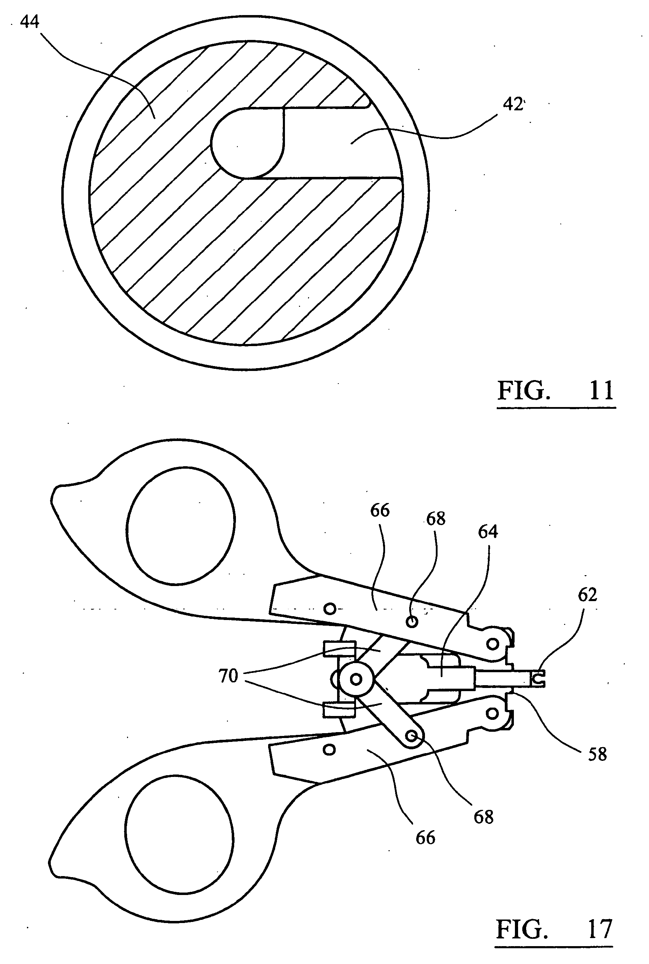

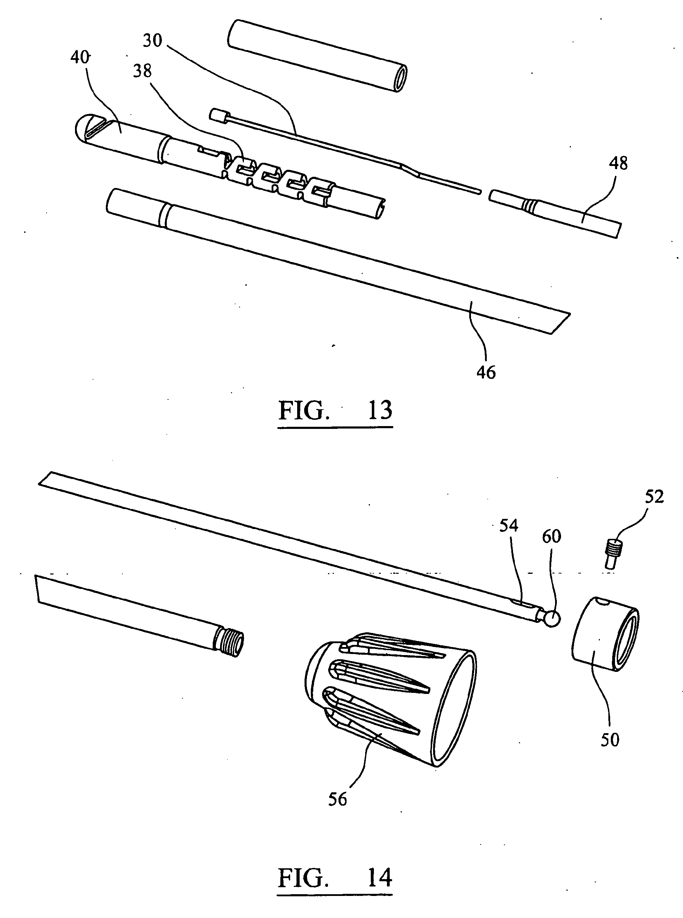

Referring back to Figures 13 to 14, cone 56 is slidably mounted on the tube 46. The sliding movement in the proximal direction is restricted by abutment with the collar. An internally threaded portion (not shown) of the cone 56 extends beyond the end of the tube and the collar whereby an external threaded portion 58 of the control mechanism shown in Figure 17 can be attached to the instrument.

The proximal end of the control rod 48 includes a reduced section leading to an enlarged head 60. The head 60 is located in a seat 62 of the control mechanism such that the rod 48 is constrained to move in the distal and proximal direction with a slider 64 in which the seat 62 is formed.

As shown in Figure 17, in order to vary the curvature of the sections a user moves two handles 66 about their respective pivots 68. That movement causes links 70 connected respectively to each handle at pivots 69 and the slider 64 to pivot about those connections to retract or extend the slider and thereby the rod 64.

Whilst the specific embodiments in the drawings show the articulated portion being inserted into a body during the surgical procedure it will be appreciated that the articulated portion may be only partially inserted or be exterior to a body during a surgical procedure.

For instance, where a number of instruments are being used, each of which is inserted through the umbilical region, it may be advantageous to enable the protruding part of at least one of the instruments to bend by using the articulated part of the instrument. This could, for instance, allow the instruments to curve away from each other in the exterior region of the umbilical regioni to make that region less crowded.

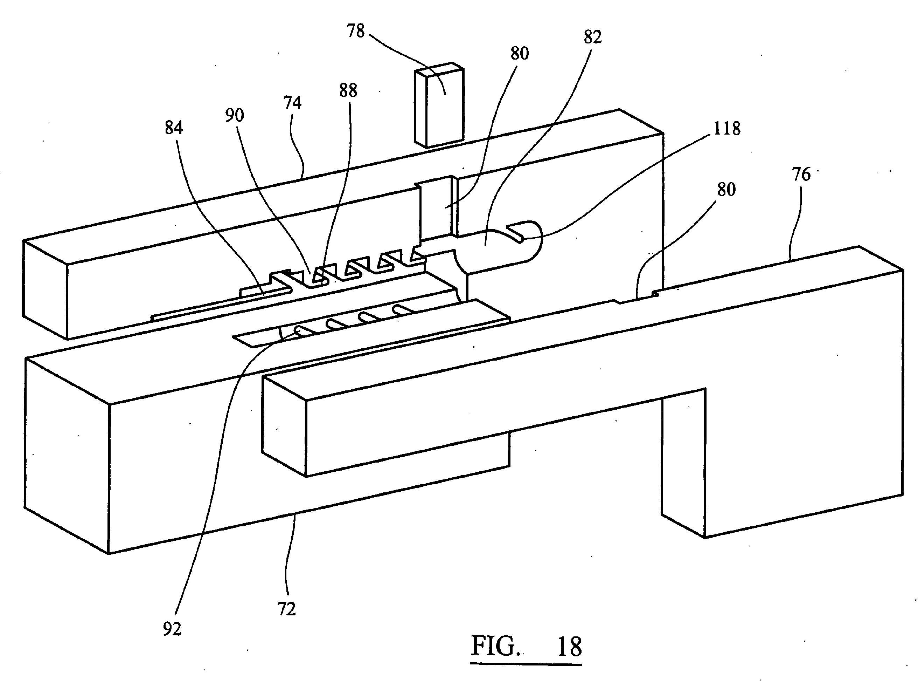

Figure 18 shows the mould that is used to make the articulated portion 13 as an integral portion.

The moulds comprise a base member 72 and and upper left and right hand members 74 and 76. In addition there is a gate member 78 that can be slotted down through opposed channels 80 in the left and right hand moulds.

In use the members 72, 74, 78 and 80 are moved towards each other to define an opening that is the shape of the articulated portion 13 shown in Figures 1A to 1B.

Each left and right hand mould has an enlarged generally semicylindrical face 82 with a forwardly and downwardly extending flange 118 that together define the slot 18.

The gate member 78 defines the recess 34 shown in Figure 10 and within which the end of the wire is held.

At the other end of the mould the left hand member alone includes a projection 84 that defines a channel above the centre line within which the control wire sits along a length axially adjacent to the articulation section. The base of that channel is at the same level as the top of the hinges 26 and the bottom of the slots 40 and 42.

The left hand mould 74 also includes projections 88 that are the same in extent and shape when viewed from the end of the mould as the projection 84. The right hand mould includes projections (not shown) that correspond to the projection 88 but which are displaced along the mould. When the moulds are brought together the opposed projections from each mould define the slots 40 and 42.

Each projection 88 on the left hand mould 74 includes a 45° trailing face 90 that cooperates with a leading face also at 45° on the other mould 72. As the moulds are brought together the inclined edges abut each other and the slots 40 and 42 are caused to have, over a very short extent, a degree of overlap. This angle may vary. The leading face of the left hand mould may also include an inclined surface arranged to cooperate with an inclined trailing face on the other mould such that the inclined faces abut each other such that the slots 40 and 42 are caused to have a slight degree of overlap. The angles of inclination may be the same.

The lower part of the projections and of the members 74 and 76 define the upper part of the hinges 26. The lower part of the hinges is defined by the upwardly extending projections 92 of the lower mould 72 that do not quite reach the top of the lower mould.

Polypropylene, such as USP Class VI grade, is injected from one end to form the articulated portion.

The polypropylene flows from one end to the other to fill the mould cavity with that flow passing through each narrow hinge 26. The hinges are 0.5 mm in depth and 1 mm in length. Preferably the maximum extent that the polypropylene has to flow through at the hinge is less than 1 mm or less than 0.75 mm. The molecules of the polypropylene become aligned as they flow along each narrow hinges to increase the strength of the hinges and to enable them to more readily resist the bending forces that are experienced in use or more able to resist stress or strain than if the machines were distributed as in the segments.

It can be seen that the moulds comprise two parts that are brought together from the side. Accordingly no rods have to be inserted longitudinally to form, for instance, the slots 40 and 42. Thus the mould is easy to form and the mould forms accurate sections of any length with any number of hinges being able to be formed accurately and consistently.

As the openings formed in the segments are visible along the complete length it is easy to thread a control member. In addition, the openings, and therefore the control member are visible along the complete length of the segments from either side. Accordingly any damage to the control member is able to be seen.

The present invention has been described in relation to a suture catcher at the end. It will be appreciated that the construction and operation of the instrument could be applied to any endoscopic instrument that requires articulation such as retractors, needle holders, cutters or searers, for example.

All of the features disclosed in this specification (including any accompanying claims, abstract and drawings), and/or all of the steps of any method or process so disclosed, may be combined in any combination, except combinations where at least some of such features and/or steps are mutually exclusive.

Each feature disclosed in this specification (including any accompanying claims, abstract and drawings) may be replaced by alternative features serving the same, equivalent or similar purpose, unless expressly stated otherwise. Thus, unless expressly stated otherwise, each feature disclosed is one example only of a generic series of equivalent or similar features.

The invention is not restricted to the details of the foregoing embodiment(s). The invention extends to any novel one, or any novel combination, of the features disclosed in this specification (including any accompanying claims, abstract and drawings), or to any novel one, or any novel combination, of the steps of any method or process so disclosed.

The scope of the invention is defined by the appended claims.

## Claims

1. An elongate portion (13) suitable for use as part of a surgical instrument (12), the elongate portion including at least two adjacent integrally formed sections (24) movable relative to each other from a first position to a second position whereby the relative direction in which a part of the elongate portion extends when compared to another part of the elongate portion is arranged to be altered by a control member (30) that extends from a proximal end of the instrument first through an opening in one section and then through an opening in another section the opening is exposed along the complete elongate extent of each section; **characterised in that** the opening (44) is at one side of the instrument at one elongate location of at least one section and at the other side (44) at a different elongate extent of that section.

2. A portion as claimed in claim 1 in which an opening (42) on a first segment that faces an adjacent second segment is visible from one side of the instrument into the opening and the opening (44) of a second segment that faces the first segment is visible from the other side.

3. A portion as claimed in any preceding claim in which the exposed opening of each section is visible from a direction perpendicular to the elongate extent of the instrument along the complete extent of each section.

4. A portion as claimed in any preceding claim in which a flexible portion (26) of reduced width connects the at least two adjacent portions which flexible portion is integrally formed with the adjacent sections.

5. A portion as claimed in claim 4 in which the flexible portion comprises a hinge.

6. A portion as claimed in claims 4 or 5 in which the flexible portion constrains two adjacent sections to move relative to each other in two opposed directions only.

7. A portion as claimed in any of claims 4 to 6 in which the at least one surface of the flexible portion of reduced width is coextensive with surfaces of adjacent segments that define at least part of the exposed openings.

8. A portion as claimed in any of claims 4 to 7 in which the molecules in the flexible portion are aligned more than the molecules in the segments whereby a greater resistance to stress is afforded.

9. A portion as claimed in any preceding claim in which the control member (30) is arranged, in use, to move at least one of adjacent sections from a straight line to a location to either side of that straight line.

10. A portion as claimed in claim 9 in which the control member is located off the centre line of the sections whereby extension of the control member in the distal direction is arranged to move the sections towards one side and retraction of the control member towards the proximal direction is arranged to move the sections towards the other side.

11. A method of moulding an elongate portion (13) as claimed in any of claims 1 to 10 suitable for use as part of a surgical instrument, the elongate portion comprising at least two adjacent integrally formed sections (24) movable relative to each other from a first position to a second position whereby the relative directions in which a part of the elongate portion extends when compared to another part of the elongate portion is arranged, in use, to be altered by a control member (30) that extends from a proximal end of the instrument first through an opening (42) in one section and then through another opening (44) in another section, the method comprising moving at least two moulds (72,74) relative to each other towards each other in a direction transverse to what will be the elongate extent of the elongate portion and injection moulding the elongate portion with the moulds causing the openings on each section to be exposed along the complete extent of each section, **characterised in that** opposed projections (90,92) from each mould are offset from each other along part of their extent whereby the moulding causes the openings of one segment to be exposed on one side along part of its extent and along the other side along a further part of the extent.

12. A method as claimed in claim 11 in which the opposed projections overlap along part of their extent whereby the openings that are exposed along each side are visible through the segment.

## Patentansprüche

1. Länglicher Abschnitt (13) zur Verwendung als Teil eines Operationsinstruments (12), wobei der längliche Abschnitt zumindest zwei benachbarte integral geformte Abschnitte (24) aufweist, die relativ zueinander aus einer ersten Stellung in eine zweite Stellung bewegt werden können, wodurch die Relativrichtung, in der sich ein Teil des länglichen Abschnitts im Vergleich zu einem anderen Teil des länglichen Abschnitts erstreckt, von einem Kontrollelement (30) verändert werden kann, das sich von einem proximalen Ende des Instruments zunächst durch eine Öffnung in einem Abschnitt und dann durch eine Öffnung in einem anderen Abschnitt erstreckt, wobei die Öffnung über die vollständige Längsausdehnung jedes Abschnitts freiliegt; **dadurch gekennzeichnet, dass** die Öffnung (44) auf einer Seite des Instruments an einer Längsstelle zumindest eines Abschnitts und auf der anderen Seite (44) an einer anderen Längsausdehnung des Abschnitts angeordnet ist.

2. Abschnitt nach Anspruch 1, in dem eine Öffnung (42) au f einem ersten Segment, das zu einem benachbarten zweiten Segment weist, von einer Seite des Instruments bis in die Öffnung zu sehen ist und in dem die Öffnung (44) eines zweiten Segments, das zum ersten Segment weist, von der anderen Seite zu sehen ist.

3. Abschnitt nach einem der vorhergehenden Ansprüche, in dem die freiliegende Öffnung jedes Abschnitts von einer Richtung senkrecht zur Längsausdehnung des Instruments über die vollständige Ausdehnung jedes Abschnitts zu sehen ist.

4. Abschnitt nach einem de r vorhergehenden Ansprüche, in dem ein flexibler Abschnitt (26) mit reduzierter Breite die zumindest zwei benachbarten Abschnitte miteinander verbindet, wobei der flexible Abschnitt einstückig mit den benachbarten Abschnitten geformt ist.

5. Abschnitt nach Anspruch 4, in dem der flexible Abschnitt ein Scharnier umfasst.

6. Abschnitt nach Anspruch 4 oder 5, in dem der flexible Abschnitt zw e i benachbarte Abschnitte festhält, damit sich diese nur in entgegengesetzten Richtungen relativ zueinander bewegen können.

7. Abschnitt nach einem der Ansprüche 4 bis 6, in dem die zumindest eine Fläche des flexiblen Abschnitts mit reduzierter Breite mit Flächen benachbarter Segmente, die zumindest einen Teil der freiliegenden Öffnungen definieren, koextensiv ist.

8. Abschnitt nach einem der Ansprüche 4 bis 7, in dem die Moleküle im flexiblen Abschnitt stärker orientiert sind als die Moleküle in den Segmenten, wodurch ein höherer Spannungswiderstand erreicht wird.

9. Abschnitt nach einem der vorhergehenden Ansprüche, in dem das Kontrollelement (30) im Gebrauch zumindest einen der benachbarten Abschnitte aus einer geraden Linie an eine Stelle auf einer der beiden Seiten der geraden Linie bewegen kann.

10. Abschnitt nach Anspruch 9, i n de m das Kontrollelement außerhalb de r Mittellinie der Abschnitte angeordnet ist, wodurch durch die Ausdehnung des Kontrollelements in distaler Richtung die Abschnitte zu einer Seite bewegt werden und bei Zurückziehen des Kontrollelements zur proximalen Richtung die Abschnitte zur anderen Seite bewegt werden.

11. Verfahren zur Formung eines länglichen Abschnitts (13) nach einem der Ansprüche 1 bis 10 zur Verwendung als Teil eines Operationsinstruments, wobei der längliche Abschnitt zumindest zwei benachbarte integral geformte Abschnitte (24) aufweist, die relativ zueinander aus einer ersten Stellung in eine zweite Stellung bewegt werden können, wodurch die Relativrichtung, in der sich ein Teil des länglichen Abschnitts im Vergleich zu einem anderen Teil des länglichen Abschni t t s erstreckt, von einem Kontrollelement (30) verändert werden kann, das sich von einem proximalen Ende des Instruments zunächst durch eine Öffnung (42) in einem Abschnitt und dann durch eine Öffnung (44) in einem anderen Abschnitt erstreckt, wobei bei dem Verfahren zumindest zwei Formen (72, 74) relativ zueinander aufeinander zu in einer Richtung quer zu der Längsausdehnung des länglichen Abschnitts bewegt werden und der längliche Abschnitt mit den Formen spritzgegossen wird, wodurch die Öffnungen auf jedem Abschnitt über die gesamte Ausdehnung jedes Abschnitts freigelegt werden, **dadurch gekennzeichnet, dass** gegenüberliegende Vorsprünge (90, 92) jeder Form entlang eines Teils ihrer Ausdehnung gegeneinander versetzt sind, wodurch die Formung dazu führt, dass die Öffnungen eines Segments auf einer Seite entlang eines Teils ihrer Ausdehnung und entlang der anderen Seite entlang eines weiteren Teils der Ausdehnung freigelegt werden.

12. Verfahren nach Anspruch 11, in dem sich die gegenüberliegenden Vorsprünge entlang eines Teils ihrer Ausdehnung überlappen, wodurch die Öffnungen, die auf jeder Seite freigelegt sind, durch das Segment sichtbar sind.

## Revendications

1. Portion (13) allongée conçue pour servir de partie dans un instrument (12) chirurgical, la portion allongée comprenant au moins deux sections (24) adjacentes formées d'un seul tenant qui sont mobiles l'une par rapport à l'autre d'une première position à une seconde position, la direction relative dans laquelle une partie de la portion allongée s'étend par rapport à une autre partie de la portion allongée étant agencée de façon à être modifiée par un organe (30) de commande qui s'étend d'une extrémité proximale de l'instrument d'abord à travers une ouverture dans une section et ensuite à travers une ouverture dans une autre section, avec l'ouverture exposée le long de l'étendue allongée complète de chaque section ; **caractérisée en ce que** l'ouverture (44) est située d'un côté de l'instrument au niveau d'un emplacement allongé d'au moins une section et de l'autre côté (44) au niveau d'une étendue allongée différente de cette section.

2. Portion selon la revendication dans laquelle une ouverture (42) sur un premier tronçon qui fait face à un second tronçon adjacent est visible depuis un côté de l'instrument dans l'ouverture et l'ouverture (44) dans un second tronçon qui fait face au premier tronçon est visible depuis l'autre côté.

3. Portion selon l'une quelconque des revendications précédentes dans laquelle l'ouverture exposée de chaque section es t visible depuis un e direction perpendiculaire à l'étendue allongée de l'instrument le long de l'étendue complète de chaque section.

4. Portion selon l'une quelconque des revendications précédentes dans laquelle une portion (26) flexible de largeur réduite relie lesdites au moins deux portions adjacentes, la portion flexible étant formée d'un seul tenant avec les sections adjacentes.

5. Portion selon la revendication 4 dans laquelle la portion flexible comporte une charnière.

6. Portion selon la revendication 4 ou 5 dans laquelle la portion flexible contraint deux sections adjacentes à se déplacer l'une par rapport à l'autre seulement dans deux directions opposées.

7. Portion selon l'une quelconque des revendications 4 à 6 dans laquelle ladite au moins une surface de la portion flexible de largeur réduite est coextensive avec les surfaces des tronçons adjacents qui délimitent au moins une partie des ouvertures exposées.

8. Portion selon l'une quelconque des revendications 4 à 7 dans laquelle les molécules dans la portion flexible sont plus alignées que les molécules dans les tronçons, ce qui permet d'obtenir une plus grande résistance à la contrainte.

9. Portion selon l'une quelconque des revendications précédentes dans laquelle l'organe (30) de commande est agencé, pendant l'utilisation, de façon à déplacer au moins une des sections adjacentes par rapport à une ligne droite vers un emplacement de chaque côté de cette ligne droite.

10. Portion selon la revendication 9 dans laquelle l'organe de commande est situé de manière décalée par rapport à la ligne centrale des sections, l'extension de l'organe de commande dans la direction distale étant agencée pour déplacer les sections vers un côté et la rétraction de l'organe de commande vers la direction proximale étant agencée pour déplacer les sections vers l'autre côté.

11. Procédé de moulage d'une portion (13) allongée selon l'une quelconque des revendications 1 à 10 conçue pour servir de partie dans un instrument chirurgical, la portion allongée comportant au moins deux sections (24) adjacentes formées d'un seul tenant qui sont mobiles l'une par rapport à l'autre d'une première position à une seconde position, les directions relatives dans lesquelles une partie de la portion allongée s'étend par rapport à l'autre partie de la portion allongée étant agencée, pendant l'utilisation, de façon à être modifiée par un organe (30) de commande qui s'étend d'une extrémité proximale de l'instrument d'abord à travers une ouverture (42) dans une section et ensuite à travers une autre ouverture (44) dans une autre section, le procédé consistant à déplacer au moins deux moules (72, 74) l'un par rapport à l'autre en les rapprochant l'un de l'autre dans une direction transversale par rapport à la direction que sera l'étendue allongée de la portion allongée et à mouler par injection la portion allongée avec les moules faisant en sorte que les ouvertures sur chaque section soient exposées le long de l'étendue complète de chaque section, **caractérisé en ce que** les saillies (90, 92) opposées depuis chaque moule sont décalées les unes par rapport aux autres le long d'une partie de leur étendue, le moulage faisant en sorte que les ouvertures d'un tronçon soient exposées sur un côté le long d'une partie de son étendue et le long de l'autre côté le long d'une autre partie de l'étendue.

12. Procédé selon la revendication 11 dans lequel les saillies opposées se chevauchent le long d'une partie de leur étendue, les ouvertures qui sont exposées le long de chaque côté étant visibles à travers le tronçon.
